(19) Europäisches Patentamt
European Patent Office
Office européen des brevets

(11) Numéro de publication: **0 039 636**
**B1**

# (12) FASCICULE DE BREVET EUROPEEN

(45) Date de publication du fascicule du brevet: **22.08.84**

(51) Int. Cl.³: **C 07 D 211/46**, C 07 D 211/22, A 61 K 31/445

(21) Numéro de dépôt: **81400670.6**

(22) Date de dépôt: **28.04.81**

(54) **Phénoxyacétates substitués d'amines cycliques, procédé d'obtention, applications à titre de médicaments et compositions pharmaceutiques les contenant.**

(30) Priorité: **30.04.80 FR 8009885**

(43) Date de publication de la demande: **11.11.81 Bulletin 81/45**

(45) Mention de la délivrance du brevet: **22.08.84 Bulletin 84/34**

(84) Etats contractants désignés: **AT BE CH DE FR GB IT LI LU NL SE**

(56) Documents cités:
**FR - A - 1 359 614**
**FR - A - 1 583 835**
**FR - A - 2 255 891**
**GB - A - 886 437**
**US - A - 3 448 110**
**US - A - 3 683 086**
**US - A - 3 708 587**
**US - A - 3 755 417**
**US - A - 4 072 754**

**CHEMICAL ABSTRACTS, vol. 67, no. 1, 03-07-1967, réf. 2990e, page 281 Columbus, Ohio, US G. GRENIER et al.: "Potential psychotropic drugs. III. Synthesis of new esters of N-methyl-3-piperidinol and 3-quinuclidinol and of new ethers of N-methyl-3-piperidinol"**
**CHEMICAL ABSTRACTS, vol. 55, no. 19, 18-09-1961, réf.**

(73) Titulaire: **ANVAR Agence Nationale de Valorisation de la Recherche, 43, rue Caumartin, F-75436 Paris Cédex 09 (FR)**

(72) Inventeur: **Godfroid, Jean-Jacques, 182, rue des Pyrénées, F-75020 Paris (FR)**
Inventeur: **Thuillier, Jean, 6, 12 rue Raffet, F-75016 Paris (FR)**

(74) Mandataire: **Phélip, Bruno et al, c/o Cabinet Harlé & Phélip 21, rue de la Rochefoucauld, F-75009 Paris (FR)**

(56) Documents cités: (suite)
**18898 b-h, Columbus, Ohio, US G. THUILLIER et al.: "Preparation of new esters and amides which act as plant growth regulators. Relation between structure and biological activity"**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des nouveaux phénoxyacétates substitués d'amines cycliques, un procédé pour leur obtention et leurs applications à titre de médicaments.

On connaît déjà des dérivés de phénoxyacétates substitués, et en particulier le 4-chloro-phénoxyacétate de diméthylaminoéthyle, qui est couramment dénommé «Méclofénoxate». Ce composé, qui est utilisé comme régulateur métabolique central, a été décrit dans le brevet FR 1 359 614 et le brevet FR «spécial de médicament» 398 M.

Dans le brevet FR 1 359 053 et le brevet FR «spécial de médicament» 463 M ainsi que dans l'article de G. THUILLIER et al. dans Bull. Soc. Chim. Fr. 1960, 1786–94, on a également décrit des régulateurs métaboliques du système nerveux central et notamment des dérivés du 4-chloro-phénoxyacétate, en particulier le 2-méthyl-4-chloro-phénoxyacétate de pipéridinoéthyle.

G. GRENIER et al. ont également décrit des phénoxyacétates et leurs propriétés psychomimétiques [Chim. Ther. 1966 (7) 408–14].

A titre de documents illustrant l'état de la technique on citera également:

– le brevet GB 866 437 qui concerne des esters basiques d'acides benzyliques éthérifiés et leurs propriétés antitussives;

– le brevet US 4 072 754 qui concerne des dérivés acides hydratropiques, à savoir les dérivés de l'acide 2-phényl-2-phénoxy-propionique, qui sont appropriés pour diminuer le taux de cholestérol;

– le brevet US 3 708 587 relatif à des acides phénoxyaliphatiques hypocholestérolémiques;

– le brevet US 3 683 086, décrit des dérivés de l'acide acétique pour le traitement de l'hyperlipémie;

– le brevet US 3 448 110 qui concerne des dérivés de l'acide isobutyrique, appropriés comme agents hypocholestérolémiques/hypolipémiques.

– le brevet FR 2 255 891 relatif à des aryloxy- et arylthioalcanes substitués et aux médicaments, doués notamment de propriétés hypocholestérolémiantes, qui renferment de tels composés.

La plupart de ces documents concernent des produits ayant des propriétés hypocholestérolémiantes ou hypolipémiantes. On notera que ces produits ont une structure aromatique très lipophile et qu'en général le carbone entre l'oxygène et la fonction ester est substitué. Ces produits sont tellement lipophiles qu'ils ne peuvent pas franchir la barrière hémato-encéphalée.

On a maintenant trouvé une nouvelle série de composés chimiques qui sont des stimulants remarquables de l'activité nerveuse centrale, capables de s'opposer aux troubles de la cellule nerveuse et de son métabolisme.

De plus, les composés de l'invention ont une activité anti-agrégante plaquettaire et une activité diurétique.

Les composés selon l'invention sont des phénoxyacétates substitués d'amines cycliques qui répondent à la formule générale:

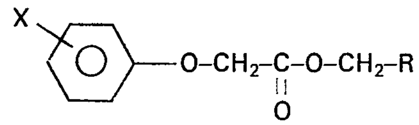

(I)

dans laquelle:

– X est le chlore, le fluor ou le radical trifluorométhyle,

– R est un groupe amino cyclique choisi parmi la pipéridine, la pyrolidine et la morpholine, ledit groupe amino étant éventuellement substitué sur l'atome d'azote par un groupe méthyle ou un groupe éthyle, l'azote dudit groupe amino étant en position 2 ou 3 par rapport au groupe $CH_2$, et les sels pharmaceutiquement acceptables desdits composés.

A titre d'exemples de groupes amino cycliques appropriés aux fins de l'invention on peut citer la pipéridine, la pyrrolidine et la morpholine.

A titre d'exemples préférés de groupes amino-cycliques qui conviennent aux fins de l'invention on citera les groupes de formules ci-après:

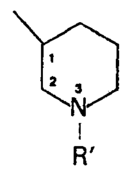

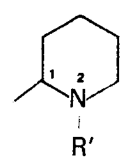

dans lesquels R' est le groupe méthyle ou éthyle.

La présente invention concerne également les sels pharmaceutiquement acceptables des composés de formule I.

L'invention a également pour objet un procédé pour l'obtention des composés de formule I. Ce procédé consiste

1) à faire réagir l'acide phénoxyacétique de formule

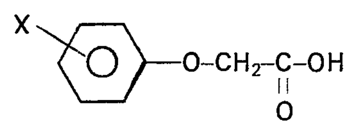

(II)

ou un de ses dérivés avec un aminoalcool cyclique de formule:

$R-CH_2-OH$ (III),

et

2) à transformer éventuellement le composé obtenu en un sel pharmaceutiquement acceptable, X, et R étant tels que définis ci-dessus.

A titre d'exemples d'aminoalcools de formule III appropriés aux fins de l'invention on citera les aminoalcools ci-après:

le N-méthyl-pipéridin-3-méthanol et le N-méthyl-pipéridine-2-méthanol.

A titre de dérivés de l'acide on peut utiliser un ester, le chlorure d'acide ou l'anhydride.

On opère avantageusement avec le chlorure de l'acide phénoxyacétique de formule II dans un

solvant hydrocarboné aliphatique ou aromatique, tel que le benzène, au reflux du solvant. De préférence, on utilise alors un excès de l'amino-alcool, celui-ci permettant de fixer l'acide chlorhydrique formé. Le produit résultant de la réaction de ce chlorure avec l'amine de formule III est donc un chlorhydrate qui peut être transformé par des moyens classiques en l'amine libre correspondante, par exemple par réaction avec un carbonate.

L'amine libre ainsi obtenue est ensuite éventuellement transformée en un sel pharmaceutiquement acceptable par réaction avec un acide couramment utilisé pour fabriquer des sels pharmaceutiquement acceptables. A titre d'exemples d'acides appropriés, on citera l'acide maléique, l'acide fumarique, l'acide oxalique, l'acide succinique et l'acide citrique.

Les composés selon l'invention ont une action régulatrice sur le système nerveux, d'origine métabolique; cette action régulatrice entraîne des modifications de la physiologie cérébrale mais aussi de la physiologie alimentaire, par un contrôle des conduites de faim et de soif. On a trouvé que les actions sur le système nerveux central de composés de l'invention sont régulatrices et stimulantes sans les caractéristiques excitantes de l'Amphétamine.

Les composés selon la présente invention conviennent pour corriger de mauvais métabolismes cérébraux et endocriniens. En particulier, les composés de l'invention peuvent être utilisés pour le traitement des obésités et des hyperphagies, des déficiences cérébrales et endocriniennes.

Enfin, l'invention concerne les compositions pharmaceutiques qui contiennent, à titre d'ingrédients actifs, un composé de formule I selon l'invention en combinaison avec un véhicule pharmaceutiquement acceptable.

Les compositions pharmaceutiques selon l'invention peuvent se présenter sous la forme de compositions administrables par voie orale ou parentérale, par exemple intraveineuse.

L'invention va être maintenant décrite plus en détail dans les exemples illustratifs ci-après. A des fins de simplification, les composés synthétisés selon l'invention sont identifiés par les abréviations PM 168, 169, 170 et 172.

Exemple 1

Préparation du chlorhydrate du (4-chlorophénoxyacétate) de N-méthyl-3-hydroxyméthylpipéridine (PM 170)

On a ajouté du chlorure d'acide parachlorophénoxyacétique (68, 33g), goutte à goutte, à une solution de 100 cm$^3$ de benzène et de N-méthylpipéridine-3-méthanol (86g) refroidie à 0 °C. Après l'addition, le mélange a été porté à reflux pendant deux heures. Après retour jusqu'à la température ordinaire, le mélange réactionnel a été filtré sur büchner, afin d'éliminer le chlorhydrate de N-méthylpipéridine-3-méthanol formé. Le filtrat a été traité dans un évaporateur rotatif afin d'éliminer le benzène et l'huile obtenue a été reprise par de l'éther. Cette solution a été lavée plusieurs fois à l'eau et la phase éthérée séchée sur sulfate de magnésium. L'éther a alors été chassé à l'évaporateur rotatif dans un bainmarie à une température de l'ordre de 60 °C afin d'éliminer les traces d'eau.

A nouveau, l'huile obtenue (70,6 g, rendement = 71,2%) a été remise en solution dans de l'éther anhydre. Dans cette solution, on a fait barboter un courant d'acide chlorhydrique sec. Le chlorhydrate a précipité et la réaction a été arrêtée après disparition de l'amine de départ et apparition du chlorhydrate. Ceci a été suivi par chromatographie sur couche mince de silice; éluant: acétate d'éthyle.

Le chlorhydrate a été soigneusement lavé à l'éther puis à l'acétone anhydres. La recristallisation a été effectuée, une fois, dans l'alcool anhydre. On a obtenu 68,4 g de PM 170; rendement: 61,4%; F = 179 °C (capillaire).

Analyse: $C_{15}H_{21}Cl_2NO_3$

| | C | H | N | Cl |
|---|---|---|---|---|
| Calculé: | 53,89 | 6,28 | 4,19 | 21,25 |
| Trouvé: | 54,15 | 6,44 | 4,31 | 21,20 |

Infra-rouge (dans KBr): fonction à 1730 cm$^{-1}$

RMN, δ (ppm), DMSOd6 pics caractéristiques: 2,72 singulet ($N^+-CH_3$); 4,17, boublet ($-O-CH_2$-pipéridine); 4,93, singulet $(O-CH_2-\underset{\underset{O}{\|}}{C}-)$.

Spectrographie de masse: ion-radical moléculaire M$^+$. à m/e 297 (334–HCl–H), à m/e 186 on note le fragment

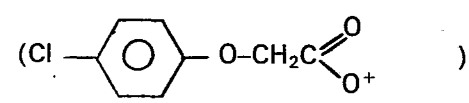

Exemple 2

On a répété le mode opératoire décrit à l'exemple 1 à la seule exception près que l'on a utilisé, à la place du N-méthyl-pipéridine-3-méthanol, l'aminoalcool ci-après:

– N-méthyl-pipéridine-2-méthanol, et on a obtenu le produit ci-après dont les caractéristiques physiques de son chlorhydrate figure dans le tableau I: le 4-chloro-phénoxyacétate de N-méthyl-2-hydroxyméthyl-pipéridine (PM 172).

Tableau 1

| N° | Point de fusion (capillaire) °C | Analyse calculé % | trouvé % | Ion-radical $M^+$ |
|---|---|---|---|---|
| PM 172 | 192 | C : 53,89<br>H : 6,28<br>N : 4,19<br>Cl : 21,25 | 54,05<br>6,39<br>4,44<br>21,37 | 297 |

Exemple 3

Essais pharmacologiques

Les composés de l'invention obtenus selon les exemples 1 et 2 ci-dessus ont été soumis à différents essais pharmacologiques et toxicologiques.

Les résultats obtenus ont été comparés avec ceux du «Méclofénoxate» qui est le 4-chlorophénoxyacétate de diméthylaminoéthyle dont la formule est la suivante:

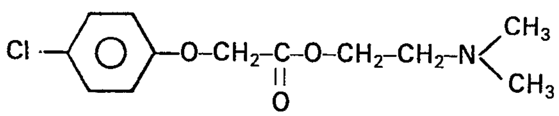 , HCl

Essai A:

Toxicité des composés de l'invention et du Méclofénoxate

Les toxicités ont été calculées par la méthode de LITCHFIELD et WILCOXON (J. Pharm. and Exp. Thérap. 1949, 96, p. 99, 113).

Les produits ont été administrés par voie orale et intraveineuse chez la souris.

Les résultats sont consignés dans le tableau II ci-après.

Tableau II
Toxicités

| Produits | DL 50 P.O. mg | DL 50 I.V. |
|---|---|---|
| Méclofénoxate | 1750 | 350 |
| PM 170 | 2300 | 175 |
| PM 172 | 1500 | 160 |

On voit que les composés de l'invention ont sensiblement tous des toxicités de même ordre de grandeur. Leur tolérance paraît bonne lorsqu'on examine les survivants sur plusieurs jours.

Le produit PM 170 est moins toxique que le «Méclofénoxate» par voie orale.

Essai B:

Actions sur le système nerveux central

1) Action sur les chromatophores du poisson

La physiologie des chromatophores de poissons sont sous la dépendance de sécrétions hypothalamiques de la partie inférieure du diencéphale. Les drogues psychotropes et à orientation cérébrale peuvent modifier la physiologie de ces régions [J. Thuillier et al. C.R. Soc. Biol. 1961, 155.10. p. 1924–1928]. On sait que ce test démontre l'action des produits sur le diencéphale qui règle la dilatation et le noircissement des chromatophores des poissons.

On utilise le Phoxinus Phoxinus Linné immergé dans un aquarium renfermant une concentration choisie de l'un des composés de l'invention préparés selon les exemples 1 et 2. On a mesuré le temps d'apparition de noircissement du poisson. On a réalisé le même test avec le Méclofénoxate. Les résultats obtenus figurent dans le tableau III ci-après.

Tableau III

| Produit | Concentration active minima | Temps d'apparition du noircissement en secondes |
|---|---|---|
| PM 170 | 0,05 pour 1000 | 90 |
| PM 172 | 0,05 pour 1000 | 60 |
| Méclofénoxate | 0,25 pour 1000 | 90 |

Les résultats du tableau III ci-dessus montrent que les composés de l'invention sont plus actifs que le «Méclofénoxate»; ils sont de 3 à 25 fois plus actifs en ce qui concerne la dose, mais ils provoquent également l'apparition plus rapide du noircissement.

2) Actions sur l'oedème cérébral du rat

Les composés alkylés de l'étain sont toxiques et provoquent un oedème sélectif du système nerveux central et en particulier du cerveau (Travaux de R. KATZMAN et al. Arch. Neurol. 9, 178, 1963). La teneur en eau du tissu cérébral est accrue. De même, le contenu cérébral en sodium augmente alors que le potassium diminue.

On a trouvé qu'à la dose de 50 mg par kilogramme, les PM 170 et 172 diminuent considérablement l'apparition de l'oedème provoqué par l'ingestion de chlorure de triéthylétain, s'opposent à la fuite du $K^+$ et limitent l'accumulation de $Na^+$. Il faut 100 mg par kg de Méclofénoxate pour obtenir le même résultat.

3) Test d'orientation après électrochoc chez le rat

Ce test consiste à provoquer une désorientation et des troubles moteurs chez le rat après répétition de 4 séries d'électrochoc. [M. Hérold: Acta. Int. Meet. Psycho. Drug. 1960].

Le PM 170 a été légèrement actif à la dose de 100 mg sur les 2 premières séries d'électrochoc.

Le Méclofénoxate est absolument inactif jusqu'à la dose de 200 mg/kg.

4) Action sur les conduites de faim

La régulation de la faim et de la soif sont sous la dépendance de stimulations sécrétrices de l'hypothalamus. Ces centres régulateurs peuvent être perturbés soit de façon acquise chez des animaux génétiquement obèses, soit à la suite de destruction des centres considérés par des poisons sélectifs, tels que l'aurothioglucose.

Tests à l'aurothioglucose chez la souris

Si l'on injecte à des souris 250 mg/kg d'aurothioglucose, les cellules des noyaux hypothalamiques dégénèrent et provoquent un syndrome d'hyperphagie chez l'animal qui se traduit par:

– une dilatation de l'estomac,

– une augmentation considérable de la croissance pondérale.

A. Action protectrice des produits de l'invention vis-à-vis de l'action de l'aurothioglucose.

Les souris ont été traitées par l'un des produits de l'invention 1/2 heure avant l'injection intrapéritonéale d'aurothioglucose (250 mg/kg).

Les produits PM 170, 172 selon l'invention ainsi administrés aux animaux s'opposent à l'hyperphagie et à la croissance pondérale des souris intoxiquées par l'aurothioglucose comme l'indiquent les résultats du tableau IV ci-après:

Tableau IV

| Code | Dose mg/kg P.O. | Nombre d'animaux | Diminution de poids de l'estomac | |
|---|---|---|---|---|
| | | | Par rapport au témoin aurothioglucose | Degré de signification |
| PM 170 | 20 | 12 | 3,2155 | P < 0,01 |
| | 50 | 12 | 2,8729 | P < 0,01 |
| | 100 | 18 | 4,2287 | P < 0,01 |
| PM 172 | 60 | 12 | 2,6299 | P < 0,02 |
| | 100 | 6 | 2,2727 | P < 0,05 |
| Méclofénoxate | 250 | 12 | 2,2495 | P < 0,05 |
| | 300 | 12 | 1,6129 | NS |

Les résultats du tableau IV montrent que le PM 170 qui, comme tous les produits de l'invention, s'oppose à la dilatation du poids de l'estomac, présente une très grande activité.

Le Méclofénoxate n'est actif qu'à la dose de 250 mg/kg, soit 12,4 fois moins actif que le PM 170.

Enfin, si l'on suit l'évolution de la courbe de croissance pondérale des souris traitées à l'aurothioglucose, on s'aperçoit que 15 jours après le début de l'expérience:

– les souris traitées à l'aurothioglucose ont un poids augmenté de 49%.

– les souris traitées à l'aurothioglucose plus Méclofénoxate ont un poids augmenté de 45%.

– les souris traitées à l'aurothioglucose plus PM 170 ont un poids augmenté de 34%.

Là encore, le Méclofénoxate est moins efficace que le PM 170 pour s'opposer à l'obésité provoquée par l'aurothioglucose.

B. Action curative du PM 170 et du Méclofénoxate après développement de l'obésité par action de l'aurothioglucose

On a rendu des souris obèses par injections intrapéritonéales d'aurothioglucose (800 mg/kg).

Après développement de l'obésité, on a obtenu un palier dans la courbe de poids. On a sélectionné alors un groupe homogène de souris et on a commencé le traitement avec le PM 170 et le «Méclofénoxate» et on a comparé les résultats obtenus avec un groupe de témoins traités uniquement à l'aurothioglucose (témoins ATG).

Le PM 170 aux doses 200, 100 et 50 mg/kg par jour pendant 18 jours, per os, a donné respectivement une chute de poids/témoins ATG de 14%, 12% et 5%.

Pour la consommation alimentaire, la diminution était de 36% à 200 mg/kg et de 33% à 100 mg/kg.

La diminution de la consommation alimentaire est de 23 à 26% respectivement pour les doses 200 et 100 mg/kg de Méclofénoxate.

En conclusion, l'activité du PM 170 est nettement supérieure au Méclofénoxate à 200 mg/kg. Il présente la même activité à 50 mg/kg que le Méclofénoxate à 200 mg/kg.

Diminution de Poids/témoins aurothioglucose:

| Dose en mg/kg | 200 | 100 | 50 |
|---|---|---|---|
| PM 170 | 14% | 12% | 5% |
| Méclofénoxate | 5% | 10% | inactif |

Baisse de consommation alimentaire/ témoins ATG:

| Dose en mg/kg | 200 | 100 | 50 |
|---|---|---|---|
| PM 170 | 36 % | 33% | 10% |
| Méclofénoxate | 22,9% | 26% | inactif |

Action des produits de l'invention sur les souris génétiquement obèses

Sur les souris génétiquement obèses, (souris pesant de 42 à 42,5 g), le produit PM 170 est efficace à la fois sur la consommation alimentaire et l'évolution pondérale comme l'indiquent les résultats du tableau V.

Tableau V

Activité du PM 170 et du Méclofénoxate sur l'obésité génétique de souris mâles et femelles

| Animaux | Produits | Consommation alimentaire | Poids |
|---|---|---|---|
| Mâles obèses traités | | 4,384 ± 0,886 | 39,3 ± 0,882 |
| Mâles obèses témoins | PM 170 | 6,786 ± 0,853 | 43,3 ± 0,284 |
| Mâles normaux traités | 200 mg/kg | 4,716 ± 0,880 | 22,22 ± 0,484 |
| Mâles normaux témoins | | 6,307 ± 0,746 | 25,34 ± 0,365 |
| Femelles obèses Traitées | Méclofénoxate | 5,542 ± 0,302 | 41,2 ± 0,802 |
| Femelles obèses témoins | 300 mg/kg | 6,347 ± 0,637 | 42,5 ± 0,787 |
| Femelles normales traitées | | 5,842 ± 0,842 | 24,8 ± 0,602 |
| Femelles normales témoins | | 6,703 ± 0,597 | 25,3 ± 0,504 |

Enfin, l'ingestion par voie gastrique pendant 18 jours de 200 mg/kg de PM 170, entraîne chez la souris obèse, une diminution régulière du poids de 8,3%, alors que dans les mêmes conditions on obtient, avec le Méclofénoxate, une diminution de poids de 2,2%.

Essai C:

Effet anti-agrégant plaquettaire

Tous les produits de l'invention préparés selon les exemples 1 et 2 à la dose de 10 mg/kg possèdent un effet anti-agrégant plaquettaire observé sur les vaisseaux pic-mériens du lapin [M.M.G. BOZEIX 7th. Int. Congress of Pharmacology, Paris, juillet 1978].

De plus, le PM 170, à la dose de 100 mg/kg, présente une légère action diurétique.

Il faut noter que le Méclofénoxate n'a pas d'effet anti-agrégant plaquettaire ni d'action diurétique.

A titre indicatif, on mentionnera que le PM 170 peut être utilisé à des doses quotidiennes de l'ordre de 0,05 à 1,00 g par jour, en prise unitaire de 0,01 à 0,25 g par forme pharmaceutique.

Les solutions injectables par voie intra-veineuse peuvent être préparées à des concentrations de 5 à 10% en poids.

On notera que parmi les composés de l'invention qui ont été testés, le PM 170 présente des propriétés remarquables; il est cinq à douze fois plus actif que le Méclofénoxate en ce qui concerne l'action de ce dernier sur le système nerveux central, et 5 à 10 fois supérieur à ce dernier quant à l'action sur les conduites de faim et de soif.

**Revendications pour les Etats contractants: BE, CH/LI, DE, FR, GB, IT, LU, NL, SE**

1.' A titre de produits nouveaux, les phénoxyacétates substitués d'amines cycliques répondant à la formule générale:

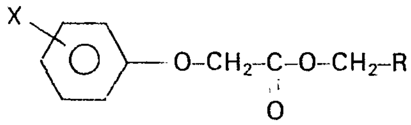

(I)

dans laquelle:

– X est le chlore, le fluor ou le radical trifluorométhyle,

– R est un groupe amino cyclique choisi parmi la pipéridine, la pyrrolidine et la morpholine, ledit groupe amino étant éventuellement substitué sur l'atome d'azote par un groupe méthyle ou un groupe éthyle, l'azote dudit groupe amino étant en position 2 ou 3 par rapport au groupe $CH_2$, et les sels pharmaceutiquement acceptables desdits composés.

2. Composé selon la revendication 1, caractérisé en ce que R est un groupe pipéridine.

3. Composé selon l'une des revendications 1 ou 2, caractérisé en ce qu'il est:

– le (4-chloro-phénoxyacétate) de N-méthyl-3-hydroxyméthylpipéridine,

– le (4-chloro-phénoxyacétate) de N-méthyl-2-hydroxyméthylpipéridine.

4. Composé selon la revendication 3, caractérisé en ce qu'il se présente sous forme de chlorhydrate.

5. Procédé pour l'obtention des composés selon l'une quelconque des revendications 1 à 4, caractérisé en ce qu'il consiste:

1) à faire réagir l'acide phénoxyacétique de formule:

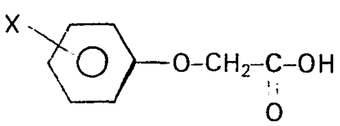

(II)

dans laquelle X est tel que défini dans la revendication 1 ou un de ses dérivés avec un aminoalcool cyclique de formule:

$R - CH_2 - OH$, R étant tel que défini dans la revendication 1 et

2) à transformer éventuellement le composé obtenu en un sel pharmaceutiquement acceptable.

6. Procédé selon la revendication 5, caractérisé en ce que l'aminoalcool cyclique est choisi parmi le N-méthyl-pipéridine-3-méthanol et le N-méthyl-pipéridine-2-méthanol.

7. Procédé selon l'une des revendications 5 ou 6, caractérisé en ce que l'étape 1) est réalisée avec le chlorure de l'acide de formule II dans un solvant aliphatique ou aromatique, tel que le benzène, au reflux du solvant.

8. Composition pharmaceutique, caractérisée en ce qu'elle contient à titre d'ingrédient actif un composé selon l'une quelconque des revendications 1 à 3 en combinaison avec un véhicule pharmaceutiquement acceptable.

9. Composition pharmaceutique selon la revendication 8, caractérisée en ce qu'elle se présente sous une forme appropriée pour une administration orale.

10. Composition pharmaceutique selon la revendication 8, caractérisée en ce qu'elle est injectable.

11. Composition pharmaceutique selon l'une des revendications 8 ou 9, caractérisée en ce qu'elle contient 0,01 à 0,25 g de (4-chloro-phénoxyacétate) de N-méthyl-3-hydroxyméthyl-pipéridine.

12. Composition pharmaceutique selon la revendication 10, caractérisée en ce qu'elle contient 5 à 10% en poids de (4-chlorophénoxyacétate) de N-méthyl-3-hydroxyméthyl-pipéridine.

**Revendications pour l'état contractant: AT**

1. Procédé de préparation de composés de formule générale

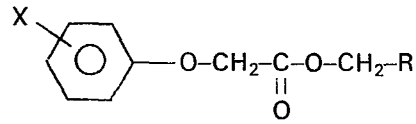

(I)

dans laquelle:

X est le chlore, le fluor ou le radical trifluorométhyle,

R est un groupe amino cyclique choisi parmi la pipéridine, la pyrrolidine, et la morpholine, ledit groupe amino étant éventuellement substitué sur l'atome d'azote par un groupe méthyle ou éthyle, l'azote dudit groupe amino étant en position 2 ou 3 par rapport au groupe $CH_2$ et les sels pharmaceutiquement acceptables desdits composés, caractérisé en ce qu'il consiste:

(1) à faire réagir l'acide phénoxyacétique de formule:

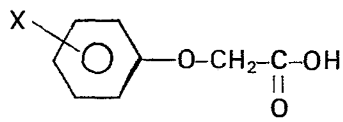

(II)

dans laquelle X est tel que défini ci-dessus ou un de ses dérivés avec un aminoalcool cyclique de formule R $CH_2$ OH, R étant tel que défini ci-dessus et

(2) à transformer éventuellement le composé obtenu en un sel pharmaceutiquement acceptable.

2. Procédé selon la revendication 1, caractérisé en ce que l'aminoalcool cyclique est choisi parmi le N-méthylpipéridine-3-méthanol et le N-méthylpipéridine-2-méthanol.

3. Procédé selon l'une des revendications 1 ou 2, caractérisé en ce que l'étape (1) est réalisée avec le chlorure de l'acide de formule II dans un solvant aliphatique ou aromatique, tel que le benzène, au reflux du solvant.

4. Procédé de préparation d'une composition pharmaceutique, caractérisé en ce que l'on combine un composé de formule I obtenu par le procédé selon l'une quelconque des revendications 1 à 3 avec un véhicule pharmaceutiquement acceptable.

5. Procédé selon la revendication 4, caractérisé en ce que le véhicule convient pour l'administration par voie orale ou par injection.

6. Procédé de préparation d'une composition pharmaceutique pour administration orale selon l'une des revendications 4 ou 5, caractérisé en ce qu'elle contient 0,01 à 0,25 g de 4-chloro-phénoxyacétate de N-méthyl-3-hydroxyméthylpipéridine.

7. Procédé de préparation d'une composition pharmaceutique injectable selon l'une des revendications 4 ou 5, caractérisé en ce qu'elle contient 5 à 10% en poids de 4-chlorophénoxyacétate de N-méthyl-3-hydroxyméthylpipéridine.

**Patentansprüche für die Vertragsstaaten: BE, CH/LI, DE, FR, GB, IT, LU, NL, SE**

1. Neue Produkte, durch zyklische Amine substituierte Phenoxyacetate, die der allgemeinen Formel entsprechen:

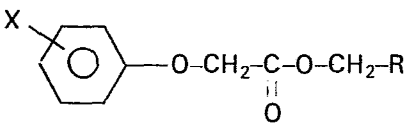 (I)

in der:

– X Chlor, Fluor oder der Trifluormethylrest ist,

– R ein zyklischer Aminorest ist, ausgewählt unter dem Piperidin-, dem Pyrrolidin- und dem Morpholinrest, wobei der erwähnte Aminorest gegebenenfalls am Stickstoffatom substituiert ist durch einen Methyl- oder einen Ethylrest, und wobei der Stickstoff des erwähnten Aminorestes in Stellung 2 oder 3 im Verhältnis zum $CH_2$-Rest ist, und die pharmazeutisch annehmbaren Salze der erwähnten Verbindungen.

2. Verbindung nach Anspruch 1, dadurch gekennzeichnet, dass R ein Piperidinrest ist.

3. Verbindung nach einem der Ansprüche 1 oder 2, dadurch gekennzeichnet, dass sie:

– das N-Methyl-3-hydroxymethylpiperidin-(4-chloro-phenoxyacetat),

– das N-Methyl-2-hydroxymethylpiperidin-(4-chloro-phenoxyacetat) ist.

4. Verbindung nach Anspruch 3, dadurch gekennzeichnet, dass sie in der Form des Chlorhydrats vorliegt.

5. Verfahren zur Gewinnung von Verbindungen nach irgendeinem der Ansprüche 1 bis 4, dadurch gekennzeichnet, dass es darin besteht:

1) die Phenoxyessigsäure nach der Formel

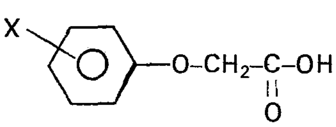 (II)

wobei X wie im Anspruch 1 definiert ist oder eines seiner Derivate mit einem zyklischen Aminoalkohol der Formel $R-CH_2-OH$, in der R die im Anspruch 1 genannte Bedeutung hat, reagieren zu lassen, und

2) die erhaltene Verbindung gegebenenfalls in ein pharmazeutisch annehmbares Salz umzuwandeln.

6. Verfahren nach Anspruch 5, dadurch gekennzeichnet, dass der zyklische Aminoalkohol ausgewählt wird unter dem N-Methyl-piperidin-3-methanol und N-Methyl-piperidin-2-methanol.

7. Verfahren nach einem der Ansprüche 5 oder 6, dadurch gekennzeichnet, dass die Stufe 1) mit dem Chlorid der Säure nach Formel II in einem aliphatischen oder aromatischen Lösungsmittel erreicht wird, wie Benzol, unter Lösungsmittelrückfluss.

8. Pharmazeutische Zusammensetzung, dadurch gekennzeichnet, dass sie als wirksamen Bestandteil eine Verbindung enthält nach gleich welchem der Ansprüche 1 bis 3 in Verbindung mit einem pharmazeutisch annehmbaren Träger.

9. Pharmazeutische Zusammensetzung nach Anspruch 8, dadurch gekennzeichnet, dass sie in einer für die orale Verabreichung geeigneten Form vorliegt.

10. Pharmazeutische Zusammensetzung nach Anspruch 8, dadurch gekennzeichnet, dass sie injizierbar ist.

11. Pharmazeutische Zusammensetzung nach einem der Ansprüche 8 oder 9, dadurch gekennzeichnet, dass sie 0,01 bis 0,25 g des N-Methyl-3-hydroxymethyl-piperidin-(4-chloro-phenoxyacetats) enthält.

12. Pharmazeutische Zusammensetzung nach Anspruch 10, dadurch gekennzeichnet, dass sie 5 bis 10 Gewichts-% des N-Methyl-3-hydroxymethyl-piperidin-(4-chloro-phenoxyacetats) enthält.

**Patentansrüche für den Vertragsstaat: AT**

1. Verfahren zur Gewinnung von Verbindungen, die der allgemeinen Formel entsprechen:

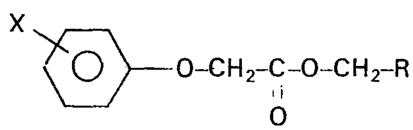 (I)

in der:

– X Chlor, Fluor oder der Trifluormethylrest ist,

– R ein zyklischer Aminorest ist, ausgewählt unter dem Piperidin-, dem Pyrrolidin- und dem Morpholinrest, wobei der erwähnte Aminorest gegebenenfalls am Stickstoffatom substituiert ist durch einen Methyl- oder einen Ethylrest, und wobei der Stickstoff des erwähnten Aminorestes in Stellung 2 oder 3 im Verhältnis zum $CH_2$-Rest ist, und die pharmazeutisch annehmbaren Salze der erwähnten Verbindungen, dadurch gekennzeichnet, dass es darin besteht:

1) die Phenoxyessigsäure nach der Formel

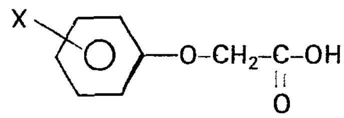 (II)

wobei X wie oben definiert ist oder eines seiner Derivate mit einem zyklischen Aminoalkohol der Formel $R-CH_2-OH$, in der R die obengenannte Bedeutung hat, reagieren zu lassen, und

2) die erhaltene Verbindung gegebenenfalls in

ein pharmazeutisch annehmbares Salz umzuwandeln.

2. Verfahren nach Anspruch 1, dadurch gekennzeichnet, dass der zyklische Aminoalkohol ausgewählt wird unter dem N-Methyl-piperidin-3-methanol und N-Methyl-piperidin-2-methanol.

3. Verfahren nach einem der Ansprüche 1 oder 2, dadurch gekennzeichnet, dass die Stufe 1) mit dem Chlorid der Säure nach Formel II in einem aliphatischen oder aromatischen Lösungsmittel erreicht wird, wie Benzol, unter Lösungsmittelrückfluss.

4. Verfahren zur Gewinnung einer pharmazeutischen Zusammensetzung, dadurch gekennzeichnet, dass man eine, durch das Verfahren nach gleich welchem der Ansprüche 1 bis 3 erhaltene Verbindung mit der allgemeinen Formel (I) mit einem pharmazeutisch annehmbaren Träger kombiniert.

5. Verfahren nach dem Anspruch 4, dadurch gekennzeichnet, dass der Träger für die orale Verabreichung oder für die Injektion geeignet ist.

6. Verfahren nach einem der Ansprüche 4 oder 5 zur Gewinnung einer, für die orale Verabreichung geeigneten pharmazeutischen Zusammensetzung, dadurch gekennzeichnet, dass sie 0,01 bis 0,25 g des N-Methyl-3-hydroxymethyl-piperidin-(4-chlorophenoxyacetats) enthält.

7. Verfahren nach einem der Ansprüche 4 oder 5 zur Gewinnung einer injizierbaren pharmazeutischen Zusammensetzung, dadurch gekennzeichnet, dass sie 5 bis 10 Gewichts-% des N-Methyl-3-hydroxymethyl-piperidin-(4-chloro-phenoxyacetats) enthält.

**Claims for the Contracting States: BE, CH/LI, DE, FR, GB, IT, LU, NL, SE**

1. Substituted phenoxyacetates of cyclic amines, corresponding to the general formula:

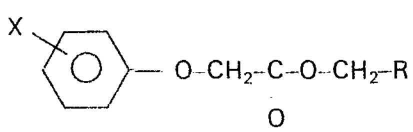 (I)

in which

X is chlorine, fluorine or the trifluoromethyl radical and

R is a cyclic amino group chosen from piperidine, pyrrolidine and morpholine, the said amino group being optionally substituted on the nitrogen atom by a methyl group or an ethyl group, and the nitrogen of the said amino group being in the 2- or 3-position relative to the $CH_2$ group, and the pharmaceutically acceptable salts of the said compounds, as new products.

2. Compound according to Claim 1, characterised in that R is a piperidine group.

3. Compound according to either of Claims 1 and 2, characterised in that it is the 4-chloro-phenoxyacetate of N-methyl-3-hydroxymethylpiperidine or the 4-chloro-phenoxyacetate of N-methyl-2-hydroxymethylpiperidine.

4. Compound according to Claim 3, characterised in that it is in the form of the hydrochloride.

5. Process for obtaining the compounds according to any one of Claims 1 to 4, characterised in that it consists in

1) reacting the phenoxyacetic acid of the formula:

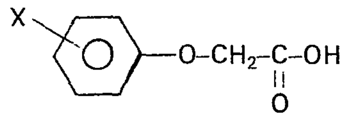 (II)

in which X is as defined in Claim 1, or one of its derivatives, with a cyclic aminoalcohol of the formula $R-CH_2-OH$, R being as defined in Claim 1, and

2) if appropriate, converting the compound obtained into a pharmaceutically acceptable salt.

6. Process according to Claim 5, characterised in that the cyclic aminoalcohol is chosen from N-methyl-piperidine-3-methanol and N-methyl-piperidine-2-methanol.

7. Process according to either of Claims 5 and 6, characterised in that stage 1) is carried out with the chloride of the acid of the formula II in an aliphatic or aromatic solvent, such as benzene, under reflux of the solvent.

8. Pharmaceutical composition, characterised in that it contains a compound according to any one of Claims 1 to 3, as the active ingredient, in combination with a pharmaceutically acceptable vehicle.

9. Pharmaceutical composition according to Claim 8, characterised in that it is in a form suitable for oral administration.

10. Pharmaceutical composition according to Claim 8, characterised in that it is injectable.

11. Pharmaceutical composition according to either of Claims 8 and 9, characterised in that it contains 0,01 to 0,25 g of the 4-chloro-phenoxyacetate of N-methyl-3-hydroxymethyl-piperidine.

12. Pharmaceutical composition according to Claim 10, characterised in that it contains 5 to 10% by weight of the 4-chloro-phenoxyacetate of N-methyl-3-hydroxymethylpiperidine.

**Claims for the Contracting State: AT**

1. Process for obtaining compounds having the structural formula:

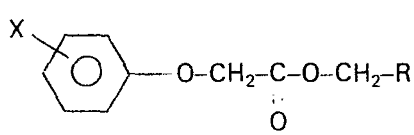 (I)

wherein:

X is chlorine, fluorine or the trifluoromethyl radical and

R is a cyclic aminogroup chosen from piperidine, pyrrolidine and morpholine, the said amino group being optionally substituted on the nitrogen atom by methyl or ethyl group, and the nitrogen of said amino group being in the 2- or 3-position relative to the $CH_2$ group and pharmaceuti-

cally acceptable salts of said compounds, characterized in that it consists in:

(1) reacting the phenoxyacetic acid of the formula:

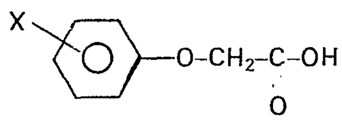

(II)

wherein X is as defined above or one of its derivatives with a cyclic aminoalcool of the formula $R-CH_2OH$, R being as defined above, and if appropriate,

(2) converting the compound obtained into a pharmaceutically acceptable salt.

2. Process according to Claim 1, characterized in that the cyclic aminoalcohol is chosen from N-methylpiperidine-3-methanol and N-methylpiperidine-2-methanol.

3. Process according to Claim 1 or 2, characterized in that stage 1 is carried out with the chloride of the acid of the formula II in an aliphatic or aromatic solvent, with reflux of the solvent.

4. Process for preparation of pharmaceutical composition characterized in that a compound of formula I, obtained by a process according to Claims 1 to 3, is combined with a pharmaceutically acceptable carrier.

5. Process according to Claim 4, characterized in that the carrier is suitable for oral administration or for injection.

6. Process according to Claims 4 or 5 characterized in that the pharmaceutical composition for oral administration comprises from 0,01 to 0,25 g of the ester 4-chloro-phenoxyacetate of N-methyl-3-hydroxymethylpiperidine.

7. Process according to Claims 4 or 5 characterized in that the pharmaceutical composition for injection comprises from 5 to 10% (W/W) of the ester 4-chloro-phenoxyacetate of N-methyl-3-hydroxymethylpiperidine.